# EUROPEAN PATENT APPLICATION

(11) **EP 1 980 199 A2**
(43) Date of publication of application: **15.10.2008**
(21) Application number: 08007242.4
(22) Date of filing: 11.04.2008
(51) Int. Cl.: A61B 5/00, H04N 5/235

(54) **Device for inspecting hollow-body cavity**

(30) Priority: 13.04.2007 JP 2007106049
(71) Applicant: Sumitomo Electric Industries, Ltd., Osaka-shi, Osaka 541-0041 (JP)
(72) Inventor: Tanaka, Masato, Yokohama-shi Kanagawa (JP); Okuno, Toshiaki, Yokohama-shi Kanagawa (JP); Hirano, Masaaki, Yokohama-shi Kanagawa (JP); Nakanishi, Tetsuya, Yokohama-shi Kanagawa (JP)
(74) Representative: HOFFMANN EITLE

(57) **Abstract**

Provided is a hollow-body cavity inspection device for inspecting a hollow-body cavity filled with a limited-wavelength transparent medium. The hollow-body cavity inspection device is capable of multifunction in addition to acquisition of an image and comprises: (1) one or more light sources for emitting two or more inspection light beams of different characteristics; (2) a light transmitting member for transmitting the two or more inspection light beams to an inspection objective in a hollow-body cavity and transmitting the reflected/scattered light from the inspection objective to the outside of the hollow-body; and (3) an inspection data formation means for receiving the reflected/scattered light and forming inspection information therefrom, wherein the two or more inspection light beams include first inspection light having its main wavelength bandwidth at the transmittable wavelength band, and the inspection data formation means comprises a plurality of means for outputting inspection information differing according to each of the two or more inspection light beams.

## Description

### BACKGROUND OF THE INVENTION

### Field of the Invention

The present invention relates to a device for optically inspecting a hollow-body cavity that is filled with a medium for which the transmittable wavelength band is limited (hereinafter, such medium is referred to as a "limited-wavelength transparent medium").

### Description of the Background Art

A known technique for optically inspecting the interior of a hollow-body cavity that is filled with a limited-wavelength transparent medium is such that light of a specific wavelength having high transmittance with respect to the medium is irradiated to the inspection objective and necessary information is obtained out of the light reflected/scattered from the inspection objective. It is impossible to obtain a desired actual image of inner wall of a vein, for example, using an endoscope and irradiating visible light into the vein, because most of the irradiated light is reflected/scattered by the blood filled in the vein. Therefore, it has been attempted to secure a necessary view range by temporarily excluding blood from an optical observation path by charging a physiological saline solution into the vein from a catheter tube or an endoscope channel, or by swelling a balloon in the vein. However, such an operation for securing a view range might damage the inside of the vein. To avoid such occurrence of damage, a known technique for acquiring an image is such that near-infrared light having high transmittance to blood is used as the irradiation light.

In a device disclosed in Japanese translation of PCT Application Publication No. 2005-507731, in order to obtain the image of a hollow-body cavity that is filled with a limited-wavelength transparent medium, a laser diode (LD) light source with a single wavelength is used such that the specific wavelength band which might be absorbed by the medium is avoided. More specifically, the light source adopted for obtaining an interior image of a vein is a LD light source that can emit near-infrared light with a single wavelength that is less absorbed by moisture and hemoglobin. And, the light with such a narrow band width is emitted from the LD light source so as to be irradiated to a target part, and the reflected/scattered light is received by an imaging device.

For obtaining a near-infrared light source, it is conceivable to use a filter to take out a near-infrared region from a wide band light source such as a halogen lamp. However, when a halogen lamp is used, the light emitted from the light source cannot efficiently be used, resulting in a failure to secure sufficient intensity of light to transmit through the medium. If the output power of the light source is increased to enhance the power of light to pass through the medium, the light energy emitted from the light source will cause an undesirable result such as unnecessary heating of a light transmitting member of the device and the surroundings.

On the other hand, even if a light source with a single wavelength that can pass through the medium is used as in the device disclosed in Japanese translation of PCT Application Publication No. 2005-507731, it will be difficult to obtain an image of the object and the surroundings if any substance that absorbs the single wavelength exists at the target part such that the reflection from the object is decreased. Also, in the case where any substances that can absorb the light having a wavelength of the LD light source to be used are intermingled with the medium, the intensity of the transmitted light will be decreased by the absorption, and accordingly it will be difficult to make an image.

In addition, with light of a single wavelength, basically only a grayscale picture can be obtained; therefore, it would be difficult to grasp the detailed visual conditions of a target part whose image is to be acquired. For example, when an image of an inner wall surface shape of a vein is to be obtained, it would be difficult to identify an alien substance and grasp the structure of the alien substance in detail, although the existence of the alien substance may be grasped with a grayscale picture. Moreover, for the purpose of inspecting the conditions of a hollow-body cavity in detail, there may be a case where simply acquiring a visual image is considered as an insufficient inspection. It will be possible to inspect a target part in more detail if the following information is obtained: inspection by spectrum analysis of the light reflected/scattered from the target part; three-dimensional shape (depth) of the target part; physical characteristics such as temperature, hardness, etc. of the target part; and the like.

### SUMMARY OF THE INVENTION

The object of the present invention is to provide a device for inspecting a hollow-body cavity filled with a limited-wavelength transparent medium, and in particular, to provide a hollow-body cavity inspection device capable of various functions in addition to acquisition of an image.

In order to achieve the object, the hollow-body cavity inspection device for optically inspecting a hollow-body cavity that is filled with a limited-wavelength transparent medium comprises:
(1) one or more light sources for emitting two or more inspection light beams of different characteristics;
(2) a light transmitting member for transmitting the two or more inspection light beams to an inspection objective in the hollow-body cavity and transmitting the reflected/scattered light from the inspection objective to the outside of the hollow-body; and
(3) an inspection data formation means for receiving the reflected/scattered light and forming inspection information therefrom. In this hollow-body cavity inspection device, the two or more inspection light beams include first inspection light whose main wavelength bandwidth lies at a transmittable wavelength band of a medium, and the inspection data formation means is equipped with a plurality of means for outputting inspection information differing according to each of the two or more inspection light beams.

The term "light beams of different characteristics" as used herein indicates that the light beams are different from each other with respect to the characteristics of light in terms of wavelength band, coherence, continuity (pulsed light or CW light), polarization state, etc. Also, the words "light beam whose main wavelength bandwidth lies at a transmittable wavelength band of a medium" as used herein indicates that the light beam has a bandwidth that includes at least one of the transmittable wavelength bands to which the medium is transparent.

### BRIEF DESCRIPTION OF THE DRAWINGS

Figure 1 is a conceptional schematic diagram showing an embodiment of hollow-body cavity inspection device relating to the present invention.
Figure 2 is a conceptional schematic diagram showing a super continuum light source.
Figures 3A and 3B are graphs showing examples of spectrum of light emitted from the super continuum light source.
Figure 4 is a conceptional schematic diagram showing a first example of a light source and an inspection data formation means in a hollow-body cavity inspection device of the present invention.
Figure 5 is a conceptional schematic diagram showing a second example of a light source and an inspection data formation means in a hollow-body cavity inspection device of the present invention.
Figures 6A and 6B are a conceptional schematic diagram showing a light source in the second example, and a graph showing the spectrum of inspection light output from the light source.
Figures 7A, 7B, and 7C are conceptional schematic diagrams showing examples of detecting physical quantity by inspection data formation means in embodiments of the present invention: Fig. 7A is detection of temperature; 7B is detection of hardness; and 7C is detection of velocity.
Figures 8A, 8B, and 8C are conceptional schematic diagrams showing depth detection means as examples of inspection data formation means in embodiments of the present invention.
Figures 9A and 9B are conceptional schematic diagrams showing, as examples of inspection data formation means in embodiments of the present invention, examples of outputting the spectral analysis information of an inspection objective or a medium.

### DETAILED DESCRIPTION OF THE INVENTION

The above-mentioned features, as well as the other features, aspects, and advantages of the present invention, will be better understood through the following description, appended claims, and accompanying drawings. In the explanation of the drawings, an identical mark is applied to identical elements and an overlapping explanation will be omitted.

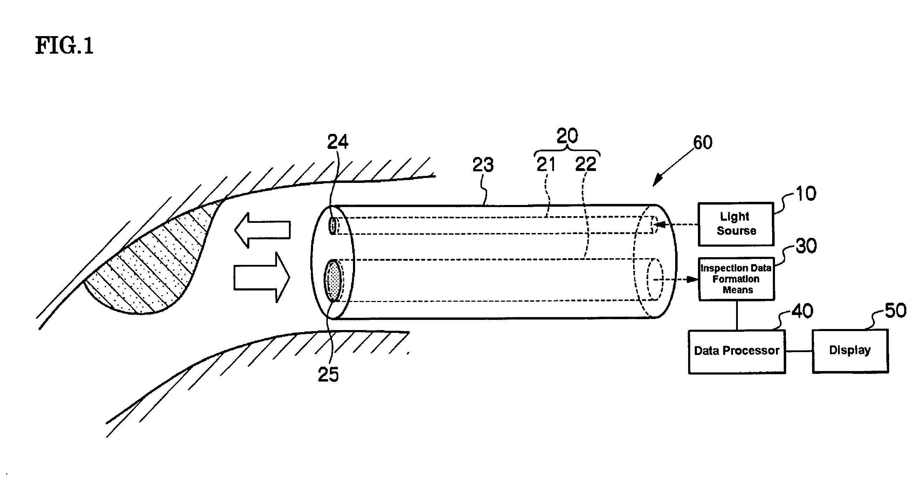

Figure 1 is a conceptional schematic diagram showing a hollow-body cavity inspection device 60 relating to an embodiment of the present invention. The hollow-body cavity inspection device 60, which is a device for optically inspecting a hollow-body cavity filled with a limited-wavelength transparent medium, is provided with a basic structure comprising a light source 10, a light transmitting member 20, and an inspection data formation means 30. Also, according to need, the hollow-body cavity inspection device is equipped with a catheter tube 23 for introducing the light transmitting member 20 into the hollow-body cavity, a data processor 40 for processing the data output from the inspection data formation means 30, and a display 50 for displaying the results of the data processing.

The light source 10 consists of one or more light sources which emit two or more inspection light beams of different characteristics. The light source 10 emits, as at least one of the two or more inspection light beams, first inspection light having the main wavelength bandwidth at the transmittable wavelength band of a medium filled in the hollow-body cavity of an inspection objective. The light transmitting member 20 is equipped with a light guide 21 for transmitting the inspection light emitted from the light source 10, and is also equipped with an image guide 22 for transmitting the light reflected/scattered from the inspection objective. An irradiation light system is provided at the tip of the light guide 21, and an objective light system 25 is provided at the tip of the image guide 22. It is also possible to use one light transmitting member as a waveguide that functions as both the light guide to transmit inspection light and the image guide to transmit the reflected/scattered light. The inspection data formation means 30 is equipped with a plurality of means for outputting different inspection information according to different inspection light. Each inspection information is processed at the data processor 40 and thereby desired inspection results are obtained. The inspection results thus obtained are displayed on the display 50.

In the case where the medium is blood, that is, when the inside of a vein is an inspection objective, the transmittable wavelength band, which is a transparent window of blood (a wavelength range where the absorption by moisture and hemoglobin less occurs), lies in the wavelength ranges of 800 to 1300 nm, 1400 to 1800 nm, and 2100 to 2400 nm. A useful light source capable of adjusting the main wavelength bandwidth to at least any of those transmittable wavelength bands is a super continuum light source (SC light source), in which narrow band light emitted from a seed light source can be made to have a broader bandwidth by passing through a nonlinear optical medium.

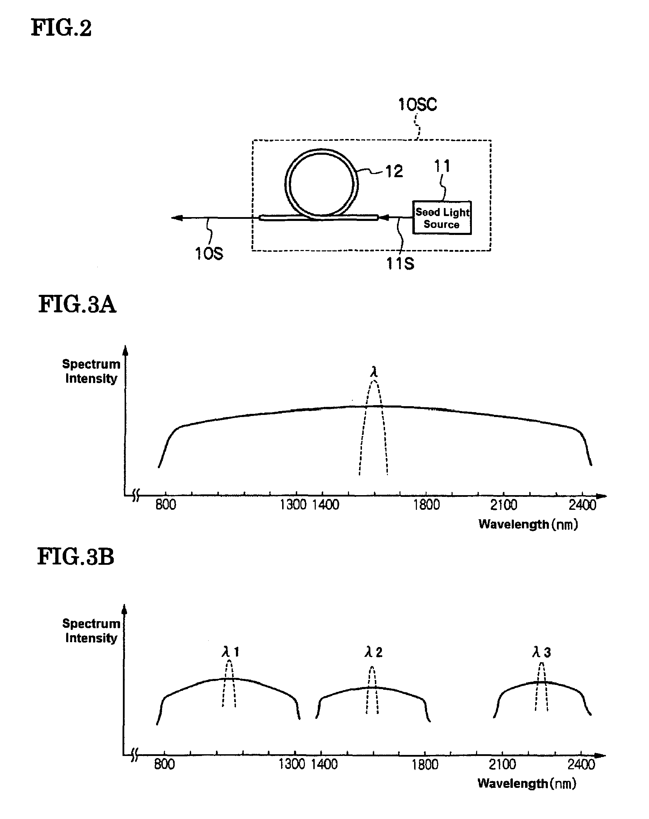

Figure 2 is a conceptional schematic diagram of a SC light source. A light source 10SC is composed of a seed light source 11 and a nonlinear optical fiber 12. Narrowband light 11s emitted from the seed light source 11 is transformed, while passing through the nonlinear optical fiber 12, into emitting light 10s having a wider bandwidth.

Figures 3A and 3B are graphs showing examples of spectrum of light emitted from the SC light source 10SC. In order to obtain, from the SC light source 10SC, light whose main wavelength bandwidth lies at the transmittable wavelength band of blood, the peak wavelength λ of the seed light source 11 is set around 1600 nm (center between 800 nm and 2400 nm) as shown in Fig. 3A. It is possible to obtain light having a bandwidth for covering the whole range of 800 to 2400 nm by appropriately adjusting the degree of bandwidth broadening due to the nonlinear optical fiber 12. Also, it is possible to obtain light having a bandwidth covering each transparent window by setting the peak wavelengths λ1, λ2, and λ3 of the seed light source 11 respectively at a position near the center of each transparent window of blood as shown in Fig. 3B.

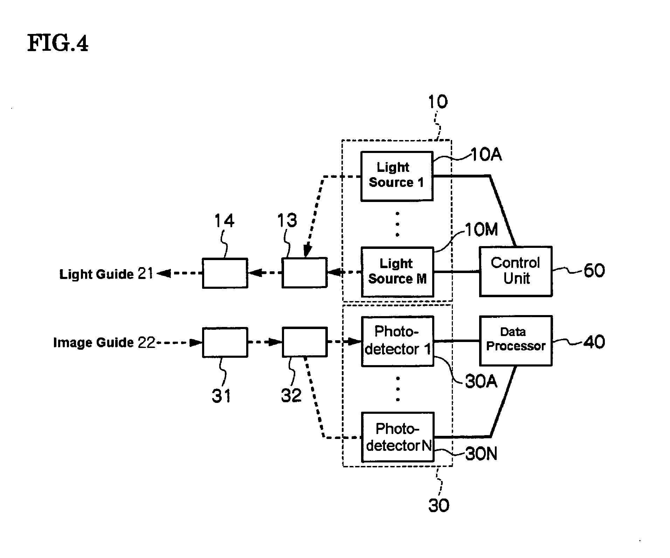

Figure 4 is a conceptional schematic diagram showing a first example for the light source 10 and the inspection data formation means 30 in the hollow-body cavity inspection device of the present invention. In the first example, the light source 10 is composed of a plurality of light sources (10A to 10M) and the inspection data formation means 30 is composed of a plurality of photodetectors (30A to 30N).

The light sources 10A to 10M each emit inspection light with differing nature, and at least one of the light sources 10A to 10M emits, as in the light source 10SC, first inspection light whose main wavelength bandwidth lies at the transmittable wavelength band of a medium. The light sources 10A to 10M are respectively controlled by a control unit 60, and may be made to emit inspection light from the light sources 10A to 10M at the same time or may be made to emit inspection light selectively from one or more of the light sources 10A to 10M. In the case where inspection light beams of different characteristics are emitted at the same time from the light source 10, the inspection light beams are multiplexed in an optical multiplexer 13, and are made incident on the light guide 21 through an optical system 14.

On the other hand, the inspection data formation means 30 is equipped with a plurality of photodetectors 30A to 30N. When the multiplexed inspection light from the light source 10 is incident on the light guide 21, the light reflected/ scattered from an inspection objective is transmitted by the image guide 22 through an optical system 31, and demultiplexed by an optical demultiplexer 32, and received respectively at the photodetector 30A to 30N. The output from the photodetectors 30A to 30N becomes different inspection information obtained from the respective inspection light of different characteristics depending on the light source 10, and the output is processed for every inspection information in a data processor 40. Also, when inspection light beams of different characteristics are selectively emitted from the light source 10, the light emitted from the image guide 22 is selectively divided into the respective photodetectors 30A to 30N, and different inspection information is obtained from the respective photodetectors 30A to 30N.

In the present invention, it is sufficient if the light source 10 can emit two or more inspection light beams of different characteristics, and it is unnecessary to provide a plurality of light sources as described in the first example. That is, the light source may be such that two or more inspection light beams of different characteristics are emitted by changing the emitting light of one light source.

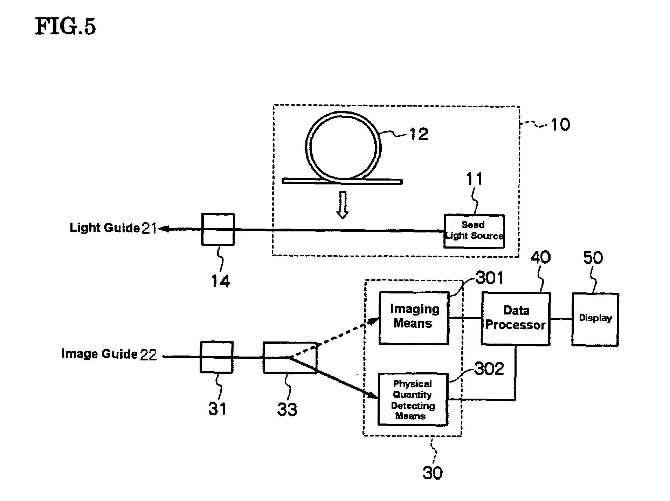

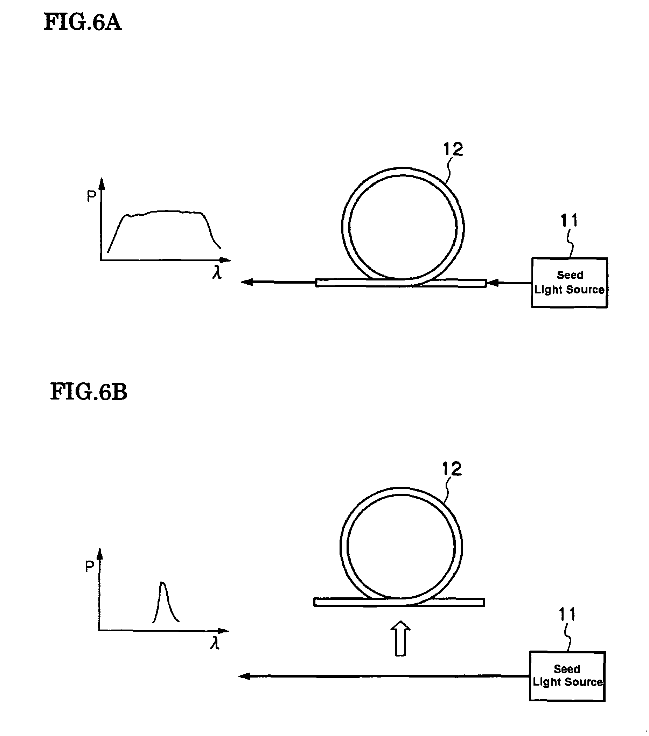

Figure 5 is a conceptional schematic diagram showing a second example of the light source 10 and the inspection data formation means 30 in the hollow-body cavity inspection device of the present invention, and Figs. 6A and 6B are conceptional schematic diagrams showing the light source 10 and graphs showing a spectrum of inspection light output from the light source 10 in the second example. In the second example, the light source 10 is composed of a seed light source 11 and a nonlinear optical fiber 12. The seed light source 11 emits narrowband light or single wavelength light. The light source 10 has two emitting modes which are interchangeable: in a first emitting mode (Fig. 6B), the narrow band light or single wavelength light is made incident, just as it is, onto the light guide 21 through the optical system 14; and in a second emitting mode (Fig. 6A), the narrowband light or single wavelength light is made incident onto the light guide 21 through the optical system 14 after having been transformed into broadband SC light by passing through the nonlinear optical fiber 12.

According to the second example, the light source 10 can emit, as the second emitting mode by means of the nonlinear optical fiber 12, at least the first inspection light whose main wavelength bandwidth lies at the transmittable wavelength band of a medium. Therefore, when optically inspecting the hollow-body cavity filled with the limited-wavelength transparent medium, it is possible to efficiently emit the light that can pass through the medium, and to obtain desired inspection information by means of reflected/scattered light from the inspection objective of the hollow-body cavity without causing undesirable problems such as heating of the surroundings, or the like. Also, even if a substance which absorbs light having a specific wavelength exists in the inspection objective, or a substance which absorbs/scatters light having a specific wavelength exists in the medium, it is possible to obtain sufficient reflected/scattered light from the hollow-body cavity surface, and to properly inspect the hollow-body cavity Particularly, when obtaining an image of a hollow-body cavity surface, reflected/scattered light can be obtained from the hollow-body cavity surface through the transparent window of the medium of the hollow-body cavity by means of inspection light having a broad bandwidth, and consequently it is possible to obtain an image having satisfactory quality and sufficient information quantity.

The light source 10 may emit inspection light beams of different characteristics by multiplexing or selectively. If inspection light is emitted in such a manner, it is possible to perform an inspection from other viewpoint by means of the inspection light of different characteristics (particularly, light that is different with respect to any of coherence, continuity, and polarization state), in addition to the inspection in which an image of a hollow-body cavity surface is obtained by means of the above-mentioned first inspection light having a bandwidth that can penetrate through the transparent window in the medium of the hollow-body cavity Accordingly, the target part of the hollow-body cavity can be inspected in more detail.

As for the inspection information forming means 30, it is equipped with an imaging means 301 for outputting the image information of an inspection objective, a physical quantity detecting means 302 for outputting the physical property information of a detected object, and a switching means 33. The imaging means 301 is, for example, a one-dimensional or two-dimensional photodetector array. The switching means 33 performs such that the light obtained from the image guide 22 through the optical system 31 is switched to the side of physical quantity detecting means 302 when the light source 10 has adopted the first emitting mode, and when the light source 10 has adopted the second emitting mode, the light obtained from the image guide 22 through the optical system 31 is switched to the side of the imaging means 301.

According to the second example, when the light source 10 adopts the first emitting mode, narrow band light or single wavelength light is irradiated to the inspection objective through the light guide 21, and light reflected/scattered from the inspection objective is acquired through the image guide 22, and then received by the physical quantity detecting means 302. Thus, when the first emitting mode is adopted, the physical property information of the inspection objective, such as temperature, hardness, flowing velocity can be obtained. On the other hand, when the light source 10 has adopted the second emitting mode, light having a broad bandwidth covering the transparent window of a medium filled in the hollow-body cavity is irradiated to the inspection objective through the light guide 21, and the light reflected/scattered from the inspection objective is acquired through the image guide 22, and then received by the imaging means 301. Thus, when the second emitting mode is adopted, the image of the hollow-body cavity surface can be obtained passing through the medium.

Also in the second example, the hollow-body cavity can be inspected in more detail, because not only can inspection information be obtained from different viewpoints, but also a satisfactory image of a hollow-body cavity can be obtained by means of wideband light that effectively uses the transparent window of the medium in the hollow-body cavity.

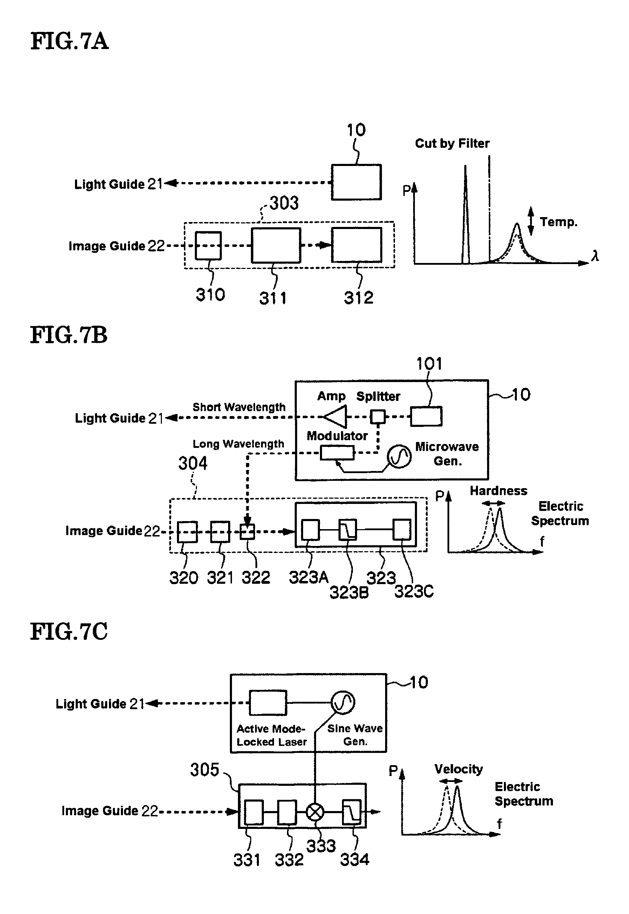

In the following, embodiments relating to the features of the light source 10 and the corresponding embodiments of the inspection data formation means 30 will be described in reference to examples. Figures 7A, 7B, and 7C are conceptional schematic diagrams showing examples of detecting physical quantity as an example of the inspection data formation means in the embodiment of the present invention; Fig. 7A shows an example of detection of temperature. In this example, a laser with single wavelength is used for the light source 10, and the temperature of a detecting object is measured using the phenomenon that the spectrum of Raman scattering changes depending on the temperature. According to the Raman scattering, light irradiated from the light source 10 generates light having a wavelength that has been shifted to the longer wavelength side than the wavelength of the irradiated light. In a temperature detecting means 303, inspection information is obtained once a photodetector 312 receives the reflected/scattered light that has been resolved into a spectrum with a spectroscope 311 after cutting of light by means of a filter 310 (such cutting is done to remove the light that has the same wavelength as the irradiated light since the Raman scattered light is minute than the irradiation light). Measuring the temperature of an inspection objective is useful as a reference for identifying an inspection objective and seeing a degree of curing progress in ablation or the like.

Figure 7B shows an example of hardness detection. In this example, Brillouin scattering is used as a means for measuring the hardness of an inspection objective. The Brillouin scattering is a phenomenon in which scattered light is generated such that the frequency is slightly shifted to the longer wavelength side relative to the original pump light, and the quantity of such frequency shift is related to hardness. The shift quantity, which is on the order of GHz, cannot be detected by a spectroscope.

Therefore, a 2-wavelength laser 101 is used as the light source 10 such that the 2-wavelength laser is capable of adjusting the frequency difference of irradiation light at the vicinity of the Brillouin frequency shift. Only short-wavelength light is irradiated at high power that will not damage an inspection objective, and the scattering light is caused to interfere with long-wavelength light. By detecting the light resulting from such interference, the Brillouin frequency shift can be measured from a spectrum of light having a difference frequency. A hardness detection means 304 comprises a wave plate 320, a long-wavelength extraction filter 321, a beam splitter 322, and a photodetector 323 which includes a photoelectric converter 323A, a low-pass filter 323B, and a spectrum measuring instrument 323C. The 2-wavelength laser 101 adopts a method in which a single laser is divided into two: one is modulated by microwave with respect to intensity and the sideband is used as long wave length light; the other one is amplified into high power by an amplifier and used as short-wavelength light. This method is advantageous in that the laser wavelength drift will not affect the results of measurement with respect to the shift quantity of the Brillouin scattering.

Figure 7C shows an example of detection of moving velocity. In this example, a light source (active mode-locked laser) used as the light source 10 is capable of oscillating periodic pulsed light by sine wave from the outside. The pulse interval of scattered light changes when the light emitted from the light source 10 hits on a scattered object having a velocity. By observing a spectrum obtained by mixing the sinusoidal wave signal that determines the pulse cycle and a signal detected with a high-speed photoelectric converter, it is possible to detect a flowing velocity because the spectral peak shifts according to the velocity. A velocity detection means 305 is equipped with a photoelectric converter 331, an amplifier 332, a mixer 333, and a low-pass filter 334. Once a flowing velocity is detected, it is possible to detect changes in the flowing velocity of blood, for example, at a part where a stenosis is caused in a vein.

In the following, an example of the inspection data formation means 30 will be described. The following example is one of a plurality of means for outputting inspection information which differs depending on each light of different characteristics, assuming that the light source 10 emits two or more light beams having different characteristics. Therefore, in the following explanation, one means for outputting inspection information corresponding to a light source having one characteristic will be described respectively. The inspection data formation means 30 constitutes an embodiment of the present invention, for example, in combination with an imaging means 301 such as shown in Fig. 5, and as for the light source 10, in combination with a light source that can emit the light whose main wavelength bandwidth lies in the transmittable wavelength band of a medium filled in the hollow-body cavity.

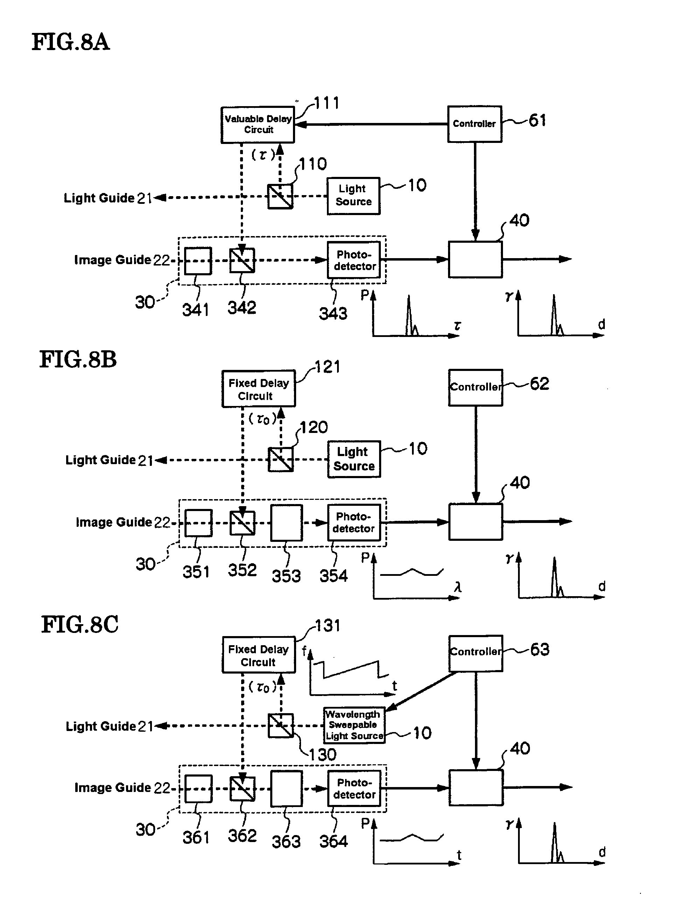

Figures 8A, 8B, and 8C are conceptional schematic diagrams each showing a depth detection means as an example of an inspection data formation means in the embodiment of the present invention. If an unevenness of an inspection objective is detected, for example, in the case of inspecting the inside of a vein, it becomes important information for seeing the conditions of the wall surface. Optical coherence tomography (OCT) is generally known as a technique for obtaining depth information with high precision.

In the case of an example shown in Fig. 8A, a wideband light source (low-coherence light source) is used as the light source 10, and the intensity of mixed light is measured while adjusting the delay (τ) of the reference light. Since the intensity of mixed light becomes maximum when the optical path difference (d) is 0, it is possible to determine the position of the reflection point of an inspection objective. That is, a variable delay circuit 111 moves in accordance with adjusting signals from a controller 61, thereby adjusting the optical path length of the reference light that is formed by dividing the light from the light source 10 at a polarized light beam splitter 110. While an inspection objective is scanned with light irradiated through the light guide 21 and the optical path length of the reference light is adjusted, the reference light is mixed, at a polarized light beam splitter 342, with the reflected/scattered light obtained through the image guide 22 and a wave plate 341, and the intensity of the mixed light thus obtained is measured at a photodetector 343. The adjusting signal of the variable delay circuit 111 is sent to the data processor 40, and the optical path length where the intensity of mixed light becomes maximum is detected, and thereby the depth of the inspection objective can be grasped at the data processor 40.

In the example shown in Fig. 8B, a wideband light source (low-coherence light source) is used as the light source 10, and the delay that is afforded to the reference light is fixed, and spectral dispersion is performed with a spectroscope after interference. In such case, a photodetector 354 having an array form is used. That is, the light emitted from the light source 10 is divided at a polarized light beam splitter 120, whereby one part becomes reference light while the other part is irradiated to an inspection objective through the light guide 21. Then, the reflected/scattered light obtained through the image guide 22 passes through a wave plate 351, and is mixed with reference light at a polarized light beam splitter 352, and then after spectral dispersion by a spectroscope 353, is received by the photodetector 354 having an array form. When the spectrum measured at the photodetector 354 is subjected to Fourier transform in the data processor 40, the characteristics thus obtained are similar to those obtained in the example of Fig. 8A.

The example shown in Fig. 8C is a modification of the example shown in Fig. 8B, and a light source that is capable of wavelength sweeping is used as the light source 10. And, similar characteristics are obtained by measuring temporal changes in the intensity of mixed light and subjecting the measurement to Fourier transform. More specifically, the light that has been subjected to wavelength sweeping is emitted from the light source 10 according to a signal from a controller 63. The light emitted from the light source 10 is divided at a polarized light beam splitter 130, and accordingly one part becomes reference light while the other part is irradiated to an inspection objective through the light guide 21. Then, the reflected/scattered light obtained through the image guide 22 passes through a wave plate 361, and is mixed with reference light at a polarized light beam splitter 362, and then after spectral dispersion by a spectroscope 363, is received by a photodetector 364 having an array form. Thus, characteristics similar to those obtained in the example of Fig. 8A can be obtained when the spectrum measured at the photodetector 364 is subjected to Fourier transform in the data processor 40 according to the signal from the controller 63.

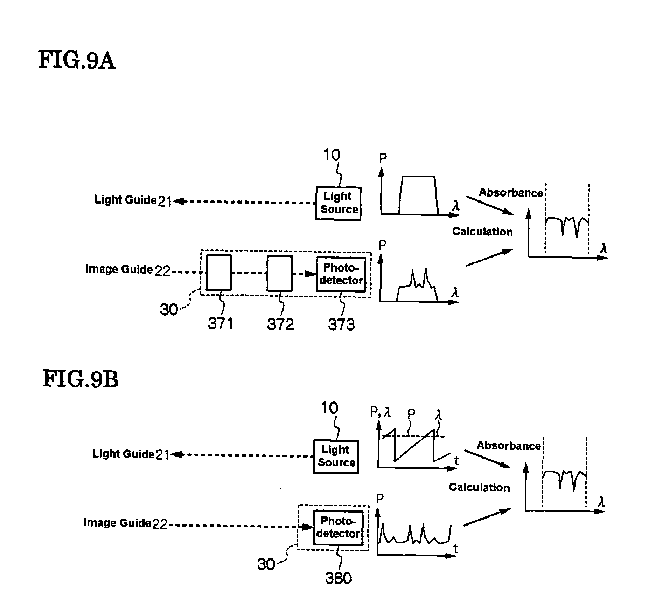

Figures 9A and 9B are conceptional schematic diagrams showing examples as an example of the inspection data formation means 30, which outputs spectral analysis information of an inspection objective or a medium. It is possible to measure the tendency of intensity thereof as a spectrum relative to the wavelength of the reflected/scattered light, and to identify components of the measured object, judging on the basis of the peak strength. Particularly, the so-called near-infrared spectroscopy, in which near-infrared light is used as one kind of light in the light source 10, is effective as a tool for analyzing a food, a medicine, and a living body.

In the example shown in Fig. 9A, the light source 10 is a light source that can output light having a specific bandwidth. Such bandwidth can be set corresponding to collected inspection information. For example, light having a bandwidth corresponding to the transparent window of blood is emitted as one characteristic of the light source 10 so that an image of vein inner wall surface may be obtained as inspection information; and at the same time, light having a bandwidth corresponding to the reflection/scattering characteristics of blood is emitted as another characteristic of the light source 10 so that the spectral analysis information of the reflected/scattered light may be obtained and thereby information on the elements of the blood may be obtained. The light emitted from the light source 10 is irradiated to an inspection objective through the light guide 21, and the reflected/scattered light obtained through the image guide 22 passes through a slit 371 and is detected by a spectrum analysis means consisting of a photodetector 373 and a spectral element 372 such as a prism and diffraction grating, etc. In this manner, spectral analysis information is output. It is possible to find the absorption characteristics of an inspection objective by performing a calculation in the data processor 40 such that the spectral analysis information obtained with the photodetector 373 is deducted from the spectral intensity of the light source 10.

In the example shown in Fig. 9B, instead of using the light source that can output light having a specific bandwidth, a light source capable of wavelength sweeping is used as the light source 10, and spectral data is obtained by synchronizing the wavelength sweeping of the light source 10 and the detection at the photodetector 380. Also, the inspection data formation means 30 may include a polarization state detection means (polarizer) which can detect changes in the polarization state of the light reflected/scattered from an inspection objective according to the emitting of linearly polarized light by the light source 10. In such case, it is possible to distinguish various elements in the inspection objective by detecting the polarization rotation that occurs due to reflection at the inspection objective; otherwise it is impossible to distinguish such various elements because of equality of reflectivity if white light is used as inspection light.

As described above, according to embodiments of the present invention, it is possible to obtain a clear image of a target part even if there exists an object that absorbs a specific wavelength in a case where it is attempted to obtain an image of a hollow-body cavity that is filled with a limited-wavelength transparent medium. Moreover, in such case, efficient use of light emitted from a light source allows the energy of the light to be prevented from heating the surroundings, or the like. Furthermore, since an inspection objective can be inspected in more detail by obtaining various kinds of information of the inspection objective, it is possible to provide a hollow-body cavity inspection device with multifunction that is not limited to acquisition of an image.

While this invention has been described in connection with what is presently considered to be the most practical and preferred embodiments, the invention is not limited to the disclosed embodiments, but on the contrary, is intended to cover all modifications and equivalent arrangements that may fall within the spirit and scope of the appended claims.

The entire disclosure of Japanese Patent Application No. 2007-106049 filed on April 13, 2007, including specification, claims, drawings, and summary, is incorporated herein in its entirety by reference.

## Claims

1. A hollow-body cavity inspection device for optically inspecting a hollow-body cavity filled with a limited-wavelength transparent medium, comprising:
one or more light sources for emitting two or more inspection light beams of different characteristics;
a light transmitting member for transmitting the two or more inspection light beams to an inspection objective in the hollow-body cavity and transmitting the reflected/scattered light from the inspection objective to the outside of the hollow-body; and
an inspection data formation means for receiving the reflected/scattered light and forming inspection information therefrom, wherein
the two or more inspection light beams include first inspection light and second inspection light, the first inspection light having its main wavelength bandwidth at the transmittable wavelength band, and wherein
the inspection data formation means comprises a plurality of means for outputting inspection information differing according to each of the two or more inspection light beams.

2. A hollow-body cavity inspection device according to claim 1, wherein the light source for emitting the first inspection light is a super continuum light source.

3. A hollow-body cavity inspection device according to claim 1, wherein the transmittable wavelength band includes any of the wavelength ranges of 800 to 1300 nm, 1400 to 1800 nm, and 2100 to 2400 nm.

4. A hollow-body cavity inspection device according to any of claims 1 to 3, wherein the light transmitting member comprises:
a light guide for transmitting the two or more inspection light beams;
an irradiation light system provided at the tip of the light guide;
an image guide for transmitting the reflected/scattered light; and
an objective light system provided at the tip of the image guide.

5. A hollow-body cavity inspection device according to any of claims 1 to 4, wherein the inspection data formation means includes an imaging means for outputting an image information of the inspection objective.

6. A hollow-body cavity inspection device according to any of claims 1 to 5, wherein the inspection data formation means includes a spectrum analysis means for outputting spectral analysis information corresponding to the second inspection light.

7. A hollow-body cavity inspection device according to any of claims 1 to 5, wherein the inspection data formation means includes a depth detection means for outputting the depth information of the inspection objective by detecting the intensity of mixed light made of the second inspection light and the reflected/scattered light.

8. A hollow-body cavity inspection device according to any of claims 1 to 5, wherein the second inspection light is light having a single wavelength, and the inspection data formation means includes a physical quantity detecting means capable of outputting the physical property information of the inspection objective according to the second inspection light.

9. A hollow-body cavity inspection device according to any of claims 1 to 5, wherein the second inspection light is pulsed light, and the inspection data formation means includes a moving velocity detecting means capable of outputting the moving velocity information of the inspection objective according to changes in the time of the reflected/scattered light pulse reaching from the inspection objective.

10. A hollow-body cavity inspection device according to any of claims 1 to 5, wherein the second inspection light is linearly polarized light, and the inspection data formation means includes a polarization state detection means for detecting changes in the polarization state of light reflected/scattered from the inspection objective.
